(19) Europäisches Patentamt
European Patent Office
Office européen des brevets

(11) EP 0 561 239 B1

(12) # EUROPÄISCHE PATENTSCHRIFT

(45) Veröffentlichungstag und Bekanntmachung des Hinweises auf die Patenterteilung:
**03.06.1998 Patentblatt 1998/23**

(51) Int Cl.[6]: **G01N 33/543**, G01N 33/58 // G01N33/94

(21) Anmeldenummer: **93103585.1**

(22) Anmeldetag: **05.03.1993**

(54) **Optischer Festphasenbiosensor auf Basis fluoreszenzfarbstoffmarkierter polyionischer Schichten**

Optical solid-phase biosensor, with fluorescence labeled polyionic layers

Biocapteur optique en phase solide, avec des couches polyioniques fluorescentes

(84) Benannte Vertragsstaaten:
**AT BE CH DE FR GB IT LI NL SE**

(30) Priorität: **18.03.1992 DE 4208645**

(43) Veröffentlichungstag der Anmeldung:
**22.09.1993 Patentblatt 1993/38**

(73) Patentinhaber: **BAYER AG**
**51368 Leverkusen (DE)**

(72) Erfinder:
- **Siegmund, Hans-Ulrich, Dr.**
  **W-4150 Krefeld 1 (DE)**
- **Heiliger, Ludger, Dr.**
  **W-5090 Leverkusen 1 (DE)**
- **van Lent, Boudewijn, Dr.**
  **W-5060 Bergisch Gladbach (DE)**
- **Becker, Arno, Dr.**
  **W-4150 Krefeld 1 (DE)**

(56) Entgegenhaltungen:
**EP-A- 0 410 323** **EP-A- 0 429 907**
**EP-A- 0 447 133** **EP-A- 0 472 990**

- **DATABASE WPIL, Woche 9032, Derwent Publications Ltd., London (GB); AN 90-242288/**
- **DATABASE WPIL, Woche 8926, Derwent Publications Ltd., London (GB); AN 89-188469/**
- **SCIENCE, Band 251, Nr. 4996, 22. Februar 1991, Washington, DC (US); BARNARD et al., Seiten 927-929/**
- **BIOMEDICINE, Band 33, Nr. 7, November 1980, Paris (FR); PACHMANN et al., Seiten 210-211/**
- **LANGONE et al., "Methods in Enzymology. Immunochemical techniques", 1981, Academic Press, New York, NY (US), Band 74, Teil C; ULLMAN et al., Seiten 28-60/**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 561 239 B1

**Beschreibung**

Die vorliegende Erfindung betrifft einen optischen Biosensor zur Detektion von Molekülen, die sich mit einem Fluoreszenzfarbstoff markieren lassen, zur Erkennung gelöster Substanzen (im folgenden Analyten genannt), für die ein diese spezifisch erkennendes Biomolekül (im folgenden Rezeptor genannt) existiert. Es handelt sich hierbei um einen Festphasensensor mit Fluoreszenzfarbstoff, der über einen Energietransferprozeß auf ein zu detektierendes, mit einem zweiten Fluoreszenzfarbstoff markiertes Molekül die Bestimmung von dessen Anwesenheit und Menge erlaubt. Über eine Verdrängungs- oder eine Sandwich- Reaktion ist die Bestimmung auch von unmarkierten Analyten möglich.

Es gibt verschiedene Methoden, Analyten, wie Hormone, Enzyme, andere Proteine, Kohlenhydrate, Nukleinsäuren, pharmakologische Wirkstoffe, Toxine und andere, in flüssigen Proben biologischen Ursprungs nachzuweisen. Unter den bekannten Methoden ragen speziell Immunoassays und damit verwandte Verfahren als eine empfindliche Nachweismethode zur Bestimmung sehr kleiner Mengen organischer Substanzen heraus. Immunoassay-Methoden beruhen allgemein auf der Fähigkeit eines Rezeptormoleküls, beispielsweise eines Antikörpers, die Struktur und molekulare Organisation eines Ligandenmoleküls, sei sie durch unpolare und/oder polare Wechselwirkungen definiert, spezifisch zu erkennen und dieses Molekül auf derartige Weise ganz spezifisch zu binden.

Immunoassays werden mit verschiedenen Methoden durchgeführt. Dazu zählen der Einsatz verschiedener Markierungstechniken, meist radioaktiver, enzymgekoppelter und auf fluoreszierender Natur (E.F. Ulman, P.L. Khanna, Methods in Enzymology, 74 (1981), 28-60). Als Sonderfall der letztgenannten Methode kann der strahlungslose Fluoreszenz-Energietransfer betrachtet werden, mit dem die Annäherung zweier Fluoreszenzfarbstoffe und darüber indirekt die Annäherung eines Rezeptor/Ligand-Paares vermessen werden kann (L. Stryer, Annual Reviews in Biochemistry 47 (1978), 819-846). In der Technik der Immunoassays und der Biosensorik hat dieses Prinzip mehrfach Erwähnung gefunden (S.M. Barnard, D.R. Walt, Science 251, 927 (1991), EP 150 905, DE 3938598).

Insbesondere bringt es Vorteile, einen Überschuß von Donorfarbstoff zu Akzeptorfarbstoff zu verwenden (DE 3938598). Dazu muß ersterer allerdings in einer entsprechend dem Förster- Radius kleiner als 5 bis 10 nm dicken, vorzugsweise monomolekularen Schicht auf einem festen Trägermaterial homogen aufgetragen sein. Dies ließ sich nach dem bisherigen Stand der Technik mittels der Langmuir-Blodgett-Technik oder mit Chemisorption erreichen. Beide Verfahren sind entweder apparativ aufwendig, unterliegen gewissen Einschränkungen bezüglich der Form der zu beschichtenden Körper oder erfordern relativ aufwendige, für die eigentliche Funktion des Sensorprinzips nicht notwendige funktionelle Gruppen an den jeweiligen aufgetragenen Molekülen. Eine Vereinfachung der Herstellbarkeit solcher dünner fluoreszierender Schichten mit vergleichsweise einfachen Molekülen ist demnach ein entscheidendes zu lösendes Problem.

In der vorliegenden Erfindung wird diese Problem dadurch gelöst, daß poly-ionische Makromoleküle, an die ein Fluoreszenzfarbstoff gebunden ist, durch rein ionische Wechselwirkungen an geladene Trägermaterialien adsorbiert werden. Die Technik der Schichterzeugung mit Polyionen ist in der deutschen Patentanmeldung DE 4 026 978 beschrieben. Überraschenderweise wurde gefunden, daß mit diesem Verfahren höhere Fluoreszenzintensitäten als mit Langmuir-Blodgett-Schichten vergleichbarer Dicke und Anzahl bei wesentlich geringerem Herstellungsaufwand, vor allem auf der apparativen Seite, zu erzielen sind.

Gegenstand der Erfindung ist ein optischer Festphasenbiosensor, markierbar mit einem Farbstoff $F_1$ und einem Rezeptormolekül zur Detektion von Analytmolekülen in flüssiger Phase, markierbar mit einem Farbstoff $F_2$ unter Ausnutzung des Försterenergietransfers zwischen $F_1$ und $F_2$, der aus

a) einem gegebenenfalls transparenten Träger,
b) einer daraufliegenden Multischicht besteht,
c) in der obersten Schicht oder einer der obersten Schichten den Fluoreszenzfarbstoff $F_1$ als Donorfarbstoff chemisch gebunden und
d) ein Antikörper oder Antigen als Rezeptor kovalent oder ionisch an die oberste Schicht gebunden enthält, an welchem
e) ein Antigen bzw. Antikörper als Analytmolekül gebunden werden kann, das wiederum mit dem Fluoreszenzfarbstoff $F_2$ markiert ist, wobei die Farbstoffmoleküle einen Abstand einnehmen, der einen strahlungslosen Försterenergietransfer ermöglicht, dadurch gekennzeichnet, daß als Multischicht b) mehrere alternierende Schichten als Polyanionen und Polykationen eingesetzt werden, und daß die Konzentration des gebundenen Analytmoleküls in Abhängigkeit von der Zunahme der relativen Fluoreszenzintensität von $F_2$ bzw. der Abnahme der Fluoreszenzintensität von $F_1$ oder der Veränderung des Verhältnisses von beiden Intensitäten gemessen wird.

Als Träger für die aufzubringenden Schichten kommen beispielsweise Gläser wie Floatglas oder Quarzglas, Halbleitermaterialien wie Silizium, Kunststoffe wie Polyester, Vinylpolymere oder Polycarbonat, sowie Metalle in Frage. Um darauf eine Verbindung über ionische Wechselwirkungen adsorbieren zu können, muß die Trägeroberfläche in ausreichendem Maße Ladungsträger aufweisen. Bei einigen der genannten Materialien, wie den Gläsern oder an der Oberfläche oxidiertem Silizium, ist dies bereits in gewissem Maße gegeben. Wenn dies nicht ausreicht, können die Trägermaterialien auch durch chemische Oberflächenmodifikation mit ionischen Gruppen belegt werden, beispielsweise mit Aminopropyl-ethoxy-dimethylsilan, wie in der deutschen Patentanmeldung DE 4 026 978 beschrieben. Auch eine oxidative Behandlung, beispielsweise durch naßchemische Oxidation, durch Koronaentladung oder durch Plasmabehandlung der Trägermaterialien, ist eine geeignete und dem Fachmann bekannte Methode, um ionisierbare Oberflächen zu erzeugen. Alternativ dazu können die Oberflächen auch Gruppen enthalten, die mit der ersten aufzubringenden Schicht chemisch unter Ausbildung kovalenter Bindungen reagieren können, wie zum Beispiel Vinylidenchlorid-Aktivierung zur Belegung mit Polyaminoverbindungen.

Als adsorbierende Polyionen werden Polyanionen und Polykationen benötigt. Als Polykationen kommen vorzugsweise Verbindungen mit Aminofunktionen in Betracht, wie Polylysin, Polyallylamin, Polyvinylpyridin, mit Aminofunktionen modifizierte Dextrane (z.B. DEAE-Dextran) sowie Chitosan. Die Aminoverbindungen können entweder durch einfache Protonierung oder durch Quaternisierung in den ionisierten Zustand überführt werden. Zu einem geringen Grade können die Aminogruppen auch mit funktionellen Gruppen, wie z.B. dem Donorfarbstoff und/oder Reaktivgruppen zur Anbindung von Rezeptormolekülen, versehen sein.

Als Polyanionen kommen vorzugsweise Polycarbonsäuren und Polysulfonsäuren in Betracht Beispiele hierfür sind Polyglutamat, Polystyrolsulfonsäure, oder Dextransulfat. Auch hier können, wie im Falle der Polykationen, funktionelle Gruppen angebracht sein. Insbesondere im Falle von Polystyrolsulfonsäure und anderen vinylischen Sulfonsäuren erweist sich der Einbau von acrylierten Cumarin-Farbstoffen oder Aminofluorescein als geeignet. Beispiele für Verbindungen dieser Art sind im Anwendungsbeispiel 1 und in der deutschen Patentanmeldung DE 4 114 482 beschrieben.

Der Aufbau einer funktionellen Multischicht erfolgt nun durch sukzessives, alternierendes Tauchen des Trägers in wäßrige Lösungen, die auch Zusätze von organischen Lösungsmitteln enthalten können, von Polyionen, z.B. Polykation, Polyanion, Polykation, u.s.f., mit dazwischen ausgeführten Spülvorgängen. Die Polyionen werden vorzugsweise in Konzentrationen zwischen 0.01 und 1 g/l im Lösungsmittel gelöst, wobei das Konzentrationsoptimum vom Molekulargewicht und der Art der verwendeten Beschichtungstechnik abhängt und im Einzelfall optimiert werden muß. Der pH der Lösung wird auf 10 oder weniger eingestellt, wobei es darauf ankommt, daß die Polyaminoverbindungen, soweit sie nicht ohnehin quaternisiert sind, in protonierter Form vorliegen und auch nicht beim nachfolgenden Eintauchen des Trägers in eine Polykationen-Lösung deprotoniert und abgelöst werden. Eine der obersten Schichten (innerhalb des Förster-Radius von 5 bis 10 nm) sollte dasjenige Polyion enthalten, an das ein Fluoreszenzfarbstoff gebunden ist. An die oberste Schicht wird dann beispielsweise ein Antikörpermolekül oder ein Antigen mit Reaktivankergruppe immobilisiert. Zur Immobilisierung von Antikörpern bieten sich die literaturbekannten Methoden und Reagenzien an, wie z. B. N-Hydroxysuccinimidester, Isocyanate und Isothiocyanate. Anwendungsbeispiel 2 zeigt, alternativ dazu, die Anbindung von aktiviertem Digitoxigenin an Polylysin.

Als Donor-/Akzeptorpaare für den Förster-Energietransfer eignen sich beispielsweise die folgenden Kombinationen von Farbstoffen:

| Donor($F_1$) | Akzeptor ($F_2$) |
|---|---|
| Fluoresceinderivate | Tetramethylrhodamin |
| Fluoresceinderivate | Texasrot |
| Cumarin der Formel I | Tetramethylrhodamin |
| Cumarin der Formel II | Tetramethylrhodamin |

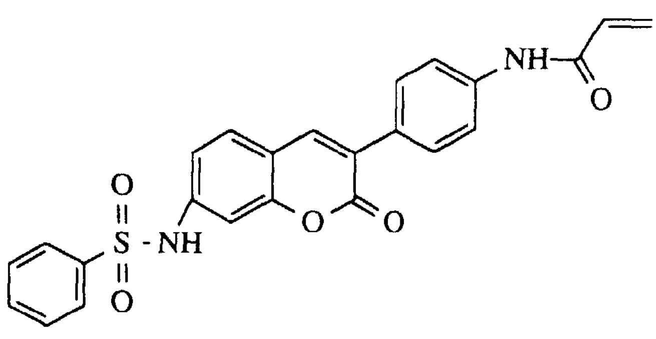

(I)

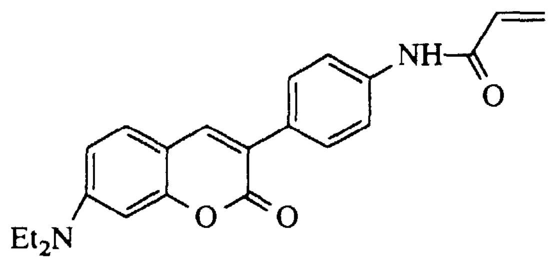

(II)

Die genannten Akzeptorfarbstoffe lassen sich sowohl mit Proteinrezeptoren als auch mit verschiedenen niedermolekularen Substanzen, sofern sie über Aminogruppen verfügen, in dem Fachmann bekannter Weise zur Reaktion bringen. An diese Bindungsstellen lassen sich anschließend mit einem zu dem in den Schichtlagen gebundenen komplementären Fluoreszenzfarbstoff ein fluoreszenzmarkierter Bindungspartner durch Förster-Energietransfer nachweisen. Beispielsweise kann an das Digitoxigenin-modifizierte Polylysin (Anwendungsbeispiel 2) ein mit Tetramethylrhodamin-isothiocyanat (TRITC) markierter monoklonaler Antikörper oder ein Fab-Fragment desselben gebunden werden. Freies Digoxin läßt sich in dieser Anordnung durch Verdrängung des Antikörpers von der Festphase oder durch vorherige Reaktion mit dem markierten Antikörper nachweisen [analog: R.G. Sommer et al., Clin. Chem. 36, 201-206 (1990)].

Der Nachweis von Analyten erfolgt durch einfaches Inkontaktbringen des beschichteten Trägers mit der Probenlösung und anschließender Fluoreszenzmessung. Ein Förster-Energietransfer läßt sich in üblichen Fluoreszenzspektrometern, aber auch in speziell dafür ausgelegten Geräten, wie z.B. in der deutschen Anmeldung DE 4 116 116 beschrieben, messen.

**Anwendungsbeispiele:**

**1. Synthese cumarinhaltiger anionischer Polymerer**

In 25 ml Dimethylsulfoxid werden folgende Substanzen gelöst:

| Polymer | A [g] | B [g] | C [g] |
|---|---|---|---|
| Natrium-p-styrylsulfonat | 2 | 3 | - |
| Kalium-sulfopropyl-methacrylat | - | - | 2,66 |
| Cumarin I | 2 | 1 | - |
| Cumarin II | - | - | 1,33 |
| Azobisiso-butyronitril | 0,02 | 0,02 | 0,02 |

Cumarin I =

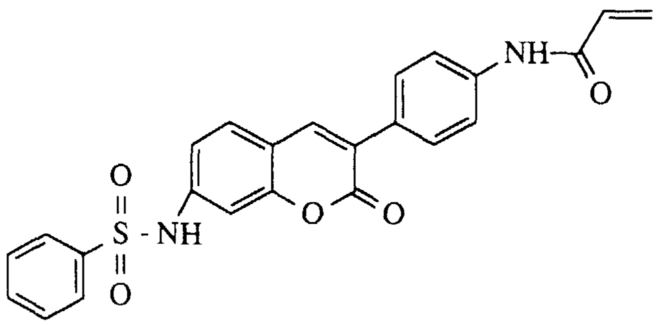

Cumarin II =

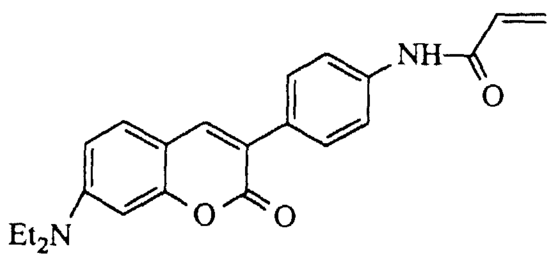

Die Lösung wird in die Polymerisationsapparatur gegeben.

Die Apparatur wird evakuiert, mit Reinststickstoff beschickt und dieser Vorgang dreimal wiederholt; die Lösung wird anschließend auf 65°C erwärmt und 15 h reagiert. Die Reaktionslösung wird in 200 ml Aceton eingetropft, der entstandene Niederschlag abfiltriert und getrocknet. Das Rohpolymer wird einer Ultrafiltration (Cutoff 10.000 Dalton) unterworfen.

Ausbeute 70 - 80 % der Theorie.

Physikalische Daten der hergestellten Polymere:,

| Fluoreszenz (gemessen in $H_2O$, pH 10) | | |
|---|---|---|
| | Polymere A, B: | Polymer C |
| $\lambda_{exc}$<br>$\lambda_{em}$ | 385 nm<br>498 nm | 410 nm<br>499 nm |

Molekulargewichte ($\overline{M}_n$, gemessen mit wäßriger Gelpermeationschromatographie):

| Polymer A | Polymer B | Polymer C |
|---|---|---|
| 150 000 | 250 000 | 180 000 |

**2. Derivatisierung von Polylysin mit Digitoxigenin**

25 mg poly-DL-Lysin werden in 15 ml Methanol gelöst und mit 20 µl Triethylamin versetzt. Unter Rühren werden bei Raumtemperatur 22 mg Digitoxigenin-(6-aminocaproyl-carboxy)-N-hydroxysuccinimid

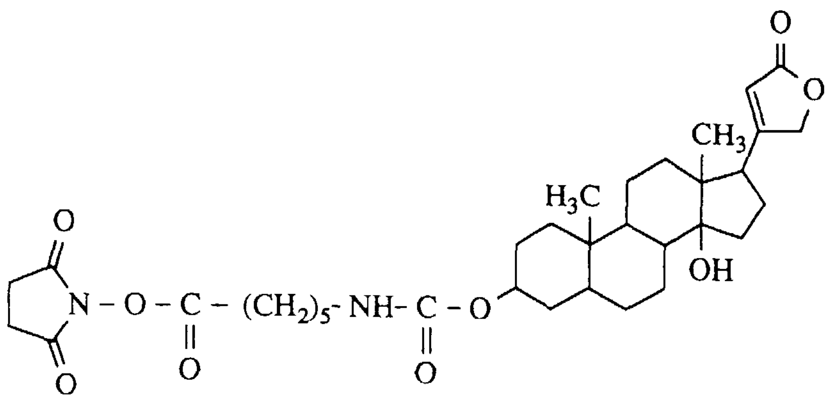

in 5 ml Isopropanol/Chloroform (1:1, v/v) zugetropft. Die Lösung wird anschließend 2 h unter Rückfluß, dann 8 h bei Raumtemperatur gerührt. Das Reaktionsgemisch wird im Vakuum eingedampft, in 2 ml Wasser aufgenommen und über eine Sephadex G-25 Säule (50 cm x 16 cm Ø, UV-Detektion 254 nm, Laufmittel: Wasser) chromatographiert. Die produkthaltigen Fraktionen werden gefriergetrocknet. Ausbeute: 27 mg (60 % d. Th.)

$^1$H-NMR (250 MHz, $CDCl_3$): 1,45 (m), 1,71 (m), 2,66 (s), 2,99 (m), 4,30 (m), 4,80 (m)ppm.

Eine immunologische Bestimmung des Digitoxigenin-Einbaus (Miles Seralyzer Digoxin Assay Kit) ergab einen Anteil Digoxinäquivalente von 2,5 Gew.-%).

**3. Reinigung und Vorbehandlung eines Glasplättchens**

**3.1. Grundreinigung**

Ein Objektträger aus Floatglas wird 1 Minute lang mit Wasser im Ultraschallbad behandelt und mit Stickstoff sorgfältig getrocknet, wobei die Oberflächen staubfrei gereinigt werden. Dann wird das Plättchen zur Vorreinigung in eine Mischung aus konz. Schwefelsäure und Wasserstoffperoxid (7:3, v/v) gebracht und darin bei 80°C eine Stunde lang im Ultraschallbad behandelt. Nach der Abkühlung auf Raumtemperatur wird das Plättchen 3 mal je 60 Sekunden in Wasser im Ultraschallbad behandelt und mit Wasser säurefrei gewaschen. Anschließend wird es in ein Gemisch aus $H_2O/H_2O_2/NH_3$ (5:1:1, v/v/v) gebracht und darin 5 Minuten lang bei 80°C behandelt. Danach wird das Plättchen sorgfältig mit Wasser salzfrei gewaschen.

**3.2. Aminosilanisierung**

Das Plättchen wird vor der Silanisierungsreaktion zur Entfernung von Wasserspuren je 2 Minuten lang in Methanol,

Methanol/Toluol (1:1, v/v) und Toluol behandelt. Danach läßt man es unter Stickstoffatmosphäre 15 h in einer 5%igen (v/v) 3-Aminopropyldimethylethoxysilan-Lösung in Toluol reagieren. Schließlich wird das Plättchen je 1 Minute lang mit Toluol, Toluol/Dimethylsulfoxid (DMSO) (1:1, v/v) und DMSO im Ultraschallbad behandelt.

**4. Herstellung eines Schichtelementes mit monomolekularer fluorophorhaltiger Lage**

Allgemeines Beschichtungsverfahren

A) Ein Probenplättchen wird 20 Minuten lang in Lösung A (Polykation) getaucht, dann 3 mal je 20 Sekunden in je 10 ml Wasser getaucht und trocknen lassen.

B) Anschließend wird 20 Minuten in Lösung B (Polyanion), wiederum 3 mal für 20 Sekunden in je 10 ml Wasser getaucht und trocknen gelassen.

4.
1. Auf Floatglas, ohne Belegung mit Aminogruppen
Die Objektträger aus Floatglas werden nach Beispiel 3.1. vorbehandelt und nach obiger Prozedur durch sukzessives Tauchen in folgende Lösungen belegt:

4.1.1.
Lösung A: 5 mg Polylysin in 10 ml 0.05 M Carbonatpuffer pH 8;
Lösung B: 5 mg Polymer C in 10 ml 0.05 M Carbonatpuffer pH 8.

4.1.2.
Lösung A: 0.5 mg DEAE-Dextran in 10 ml 0.05 M Carbonatpuffer pH 8;
Lösung B: 0.5 mg Polymer C in 10 ml 0.05 M Carbonatpuffer pH 8.

4.1.3.
Lösung A: 10 mg Polylysin in 10 ml 0.05 M Carbonatpuffer pH 8;
Lösung B: 1 mg Polymer C in 10 ml 0.05 M Carbonatpuffer pH 8.

4.1.4.
Lösung A: 1 mg DEAE-Dextran in 10 ml 3 mM Salzsäure;
Lösung B: 1 mg Polymer C in 10 ml 3 mM Salzsäure.

4.1.5.
Lösung A: 0.1 mg Polyvinylpyridin (Reilline 2200) in 10 ml 3 mM Salzsäure;
Lösung B: 10 mg Polymer A in 10 ml 3 mM Salzsäure.

4.2. Auf Floatglas, mit Belegung mit Aminogruppen

Die Objektträger aus Floatglas werden nach Beispiel 3.2. vorbehandelt und in Abwandlung der allgemeinen Prozedur lediglich durch Tauchen in Lösung B belegt. Es lassen sich die Lösungen von 4.1.1. bis 4.1.4 verwenden.

4.3. Auf Polyesterfolie

Ein Stück von mit Vinylidenchlorid oberflächenaktivierter Polyesterfolie wird ohne weitere Reinigungsschritte analog der obigen allgemeinen Prozedur beschichtet. Es lassen sich die Lösungen von 4.1.1. bis 4.1.4. verwenden.

**5. Messung der Bindung von TRITC-markiertem Anti-Digoxin-IgG an das Schichtelement**

Ein nach Beispiel 4.1.3. beschichteter Träger wird daran anschließend durch Tauchen in eine Lösung von 0.1 mg/ml Digitoxigenin-derivatisiertem Polylysin (Beispiel 2) und Waschen gemäß der allgemeinen Prozedur mit einer spezifisch bindenden Schicht überzogen.
Das Plättchen wird anschließend mit 50 µl einer Lösung von TRITC-Anti-Digoxin-IgG (hergestellt durch Reaktion von Tetramethylrhodamin-isothiocyanat und Anti-Digoxin-IgG) in Kontakt benetzt (Proteinkonzentration 0.1 mg/ml). Nach Spülen mit einem Puffer (15 mM $KH_2PO_4$, 25 mM Citrat, 1 mM $MgCl_2$, 50 mM KCl, 0.4 g/l $NaN_3$, 1 g/l Rinderserumalbumin, pH 6.4) wird die Fluoreszenz nochmals gemessen, wobei die des Cumarins aus dem Polymer C (509 nm)

zugunsten der Fluoreszenz des TRITC (587 nm) gelöscht wird (Figur 1, Kurve a). Zur Kontrolle wird anstelle des Digitoxigenin-derivatisierten Polylysin underivatisiertes Polylysin in gleicher Konzentration eingesetzt, wodurch die oberste Schicht nicht zur Bindung befähigt ist und sich kein Energietransfer ergibt (Figur 1, Kurve b).

**Patentansprüche**

1. Optischer Festphasenbiosensor, markierbar mit einem Farbstoff $F_1$ und einem Rezeptormolekül zur Detektion von Analytmolekülen in flüssiger Phase, markierbar mit einem Farbstoff $F_2$ unter Ausnutzung des Försterenergietransfers zwischen $F_1$ und $F_2$, der aus

   a) einem gegebenenfalls transparenten Träger,

   b) einer daraufliegenden Multischicht besteht,

   c) in der obersten Schicht oder einer der obersten Schichten den Fluoreszenzfarbstoff $F_1$ als Donorfarbstoff chemisch gebunden und

   d) ein Antikörper oder Antigen als Rezeptor kovalent oder ionisch an die oberste Schicht gebunden enthält, an welchem

   e) ein Antigen bzw. Antikörper als Analytmolekül gebunden werden kann, das wiederum mit dem Fluoreszenzfarbstoff $F_2$ markiert ist, wobei die Farbstoffmoleküle einen Abstand einnehmen, der einen strahlungslosen Försterenergietransfer ermöglicht, dadurch gekennzeichnet, daß als Multischicht b) mehrere alternierende Schichten als Polyanionen und Polykationen eingesetzt werden, und daß die Konzentration des gebundenen Analytmoleküls in Abhängigkeit von der Zunahme der relativen Fluoreszenzintensität von $F_2$ bzw. der Abnahme der Fluoreszenzintensität von $F_1$ oder der Veränderung des Verhältnisses von beiden Intensitäten gemessen wird.

2. Optischer Biosensor gemäß Anspruch 1, in dem das Analytmolekül e) ein mit dem Fluoreszenzfarbstoff $F_2$ markiertes, an die oberste Schicht gebundenes Molekül, das nicht der Analyt ist, von der obersten Schicht verdrängt und die Konzentration des schließlich gebundenen Analytmoleküls in Abhängigkeit von der Abnahme der relativen Fluoreszenzintensität von $F_2$ bzw. der Zunahme der Fluoreszenzintensität von $F_1$ oder der Änderung des Verhältnisses von beiden Intensitäten gemessen wird.

3. Optischer Biosensor gemäß Anspruch 1 oder 2, in dem als Trägermaterial a) Floatglas, Quarzglas, Silizium, Polyester, Vinylpolymere oder Polycarbonat eingesetzt werden.

4. Optischer Biosensor nach Anspruch 1 oder 2, bei dem es sich bei den polyanionischen Verbindungen um Polyglutaminsäure, Polystyrolsulfonsäure, Dextransulfat oder ein Copolymeres aus Styrolsulfonsäure oder anderen vinylischen Sulfonsäuren und einem Vinylderivat eines Fluoreszenzfarbstoffes handelt.

5. Optischer Biosensor nach Anspruch 4, bei dem es sich bei den polyanionischen Verbindungen um Copolymere aus Styrolsulfonat oder Sulfopropylmethacrylat und dem Acrylat eines Cumarinfarbstoffs handelt.

6. Optischer Biosensor nach Anspruch 1 oder 2, bei dem es sich bei den polykationischen Verbindungen um Polylysin, Polyallylamin, Polyvinylpyridin, Chitosan oder deren durch Permethylierung quaternisierte Ammoniumsalze, oder um DEAE-Dextran handelt.

7. Optischer Biosensor nach Anspruch 6, bei dem es sich bei den polykationischen Verbindungen um teilweise mit Fluorescein-isothiocyanat oder einem anderen Reaktivfarbstoff derivatisierte Polyaminoverbindungen wie Polylysin oder Polyallylamin handelt.

8. Optischer Biosensor nach Anspruch 1 oder 2, dadurch gekennzeichnet, daß als für einen Förstertransfer geeignetes Farbstoffpaar $F_1$ und $F_2$

   Fluoresceinderivate und Tetram ethylrhodamin,

Fluoresceinderivate und Texasrot,

Cumarin der Formel I und Tetramethylrhodamin

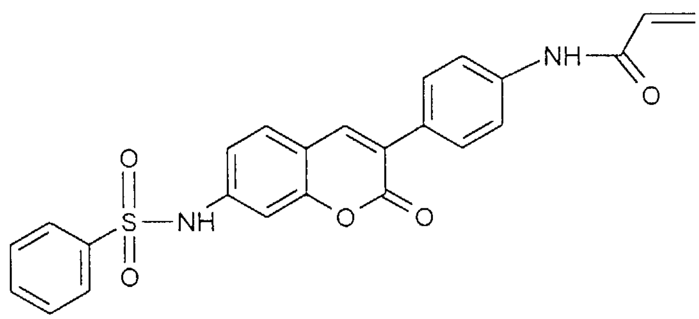

(I)

oder

Cumarin der Formel II und Tetramethylrhodamin,

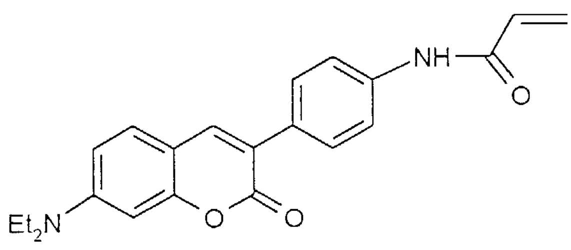

(II)

eingesetzt werden.

**9.** Optischer Biosensor nach Anspruch 8, in dem

Cumarin der Formel I und Tetramethylrhodamin
oder

Cumarin der Formel II und Tetramethylrhodamin

als Farbstoffpaar $F_1$ und $F_2$ eingesetzt werden.

**10.** Verfahren zur Bestimmung von Digoxin als Analytmolekül mit einem Biosensor gemäß Anspruch 1, dadurch gekennzeichnet, daß auf einen Glasträger als erste Schicht ein Polylysin, als zweite Schicht ein Copolymer aus Kaliumsulfopropylmethacrylat und einem Cumarin der Formel II als Farbstoff $F_1$

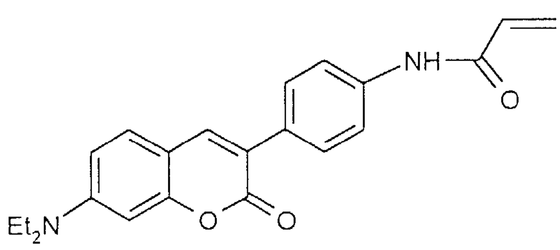

(II)

und als oberste Schicht ein Polylysin, derivatisiert mit Digitoxigenin als Rezeptor, aufgetragen wird, Anti-Digoxin-Immunglobulin G mit Tetramethylrhodaminisothiocyanat als Farbstoff $F_2$ in Lösung markiert wird, der Sensor in die Lösung eingetaucht wird, anschließend in eine Digitoxinhaltige Lösung eingetaucht wird und die Abnahme der Fluoreszenzintensität aus dem Farbstoff $F_2$ bzw. die Zunahme der Fluoreszenzintensität aus dem Farbstoff $F_1$ oder die Änderung des Verhältnisses von beiden gemessen wird.

**11.** Verfahren zur Herstellung eines optischen Biosensors gemäß Anspruch 1 oder 2, dadurch gekennzeichnet, daß auf einem gegebenenfalls transparenten Träger durch sukzessives alternierendes Eintauchen des Trägers in Lösungen von polyanionischen und polykationischen Verbindungen mehrere Schichten aufgetragen werden, der Träger nach jedem Schichtauftrag gewaschen und getrocknet wird, in dem eine der obersten Polyionen-Schichten einen fluoreszenzfähigen Farbstoff $F_1$ chemisch gebunden enthält und die oberste Schicht einen Rezeptor kovalent oder ionisch gebunden enthält.

**12.** Verwendung des optischen Biosensors nach Anspruch 1 oder 2 zur Detektion biochemisch aktiver Verbindungen.

**Claims**

**1.** Optical solid-phase biosensor which can be labelled with a dye $F_1$ and with a receptor molecule for detecting analyte molecules in liquid phase, which can be labelled with a dye $F_2$ utilizing the Förster energy transfer between $F_1$ and $F_2$, which consists of

a) an optionally transparent substrate,
b) a multilayer located thereon,
c) contains in the top layer or one of the top layers the fluorescent dye $F_1$ as donor dye chemically bonded and
d) an antibody or antigen as receptor covalently or ionically bonded to the top layer, to which receptor
e) an antigen or antibody, respectively, can be bound as analyte molecule which, in turn, is labelled with the fluorescent dye $F_2$, where the dye molecules are located at a distance which permits radiationless Förster energy transfer, characterized in that a plurality of alternating layers of polyanions and polycations are employed as multilayer b), and in that the concentration of the bound analyte molecule is measured as a function of the increase in the relative fluorescence intensity of $F_2$ or the decrease in the fluorescence intensity of $F_1$ or the change in the ratio of the two intensities.

**2.** Optical biosensor according to Claim 1, in which the analyte molecule e) displaces a molecule which is labelled with the fluorescent dye $F_2$ and is bound to the top layer and which is not the analyte from the top layer, and the concentration of the eventually bound analyte molecule is measured as a function of the decrease in the relative fluorescence intensity of $F_2$ or the increase in the fluorescence intensity of $F_1$ or the change in the ratio of the two intensities.

**3.** Optical biosensor according to Claim 1 or 2, in which float glass, quartz glass, silicon, polyester, vinyl polymers or polycarbonate are employed as substrate material a).

**4.** Optical biosensor according to Claim 1 or 2, in which the polyanionic compounds are polyglutamic acid, polystyrenesulphonic acid, dextran sulphate or a copolymer of styrenesulphonic acid or other vinylic sulphonic acids and a vinyl derivative of a fluorescent dye.

**5.** Optical biosensor according to Claim 4, in which the polyanionic compounds are copolymers of styrenesulphonate or sulphopropyl methacrylate and the acrylate of a coumarin dye.

**6.** Optical biosensor according to Claim 1 or 2, in which the polycationic compounds are polylysine, polyallylamine, polyvinylpyridine, chitosan or their ammonium salts quaternized by permethylation, or DEAE-dextran.

**7.** Optical biosensor according to Claim 6, in which the polycationic compounds are polyamino compounds, such as polylysine or polyallylamine, partly derivatized with fluorescein isothiocyanate or another reactive dye.

**8.** Optical biosensor according to Claim 1 or 2, characterized in that the pair of dyes $F_1$ and $F_2$ employed as suitable for a Förster transfer are fluorescein derivatives and tetramethylrhodamine, fluorescein derivatives and texas red, coumarin of the formula I and tetramethylrhodamine

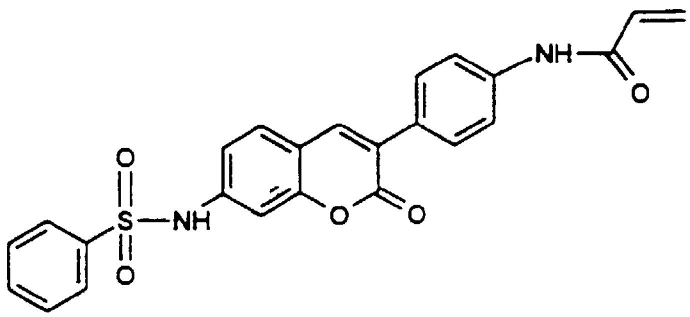

(I)

or coumarin of the formula II and tetramethylrhodamine

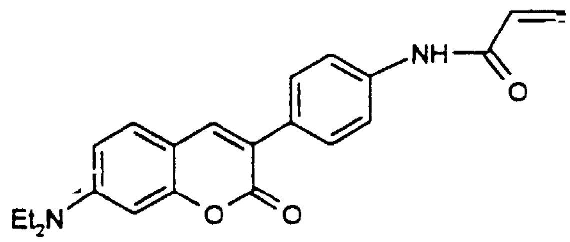

(II)

**9.** Optical biosensor according to Claim 8, in which

coumarin of the formula I and tetramethylrhodamine or
coumarin of the formula II and tetramethylrhodamine are employed as pair of dyes $F_1$ and $F_2$.

**10.** Method for determining digoxin as analyte molecule using a biosensor according to Claim 1, characterized by application to a glass substrate of a polylysine as first layer, of a copolymer of potassium sulphopropyl methacrylate and a coumarin of the formula II as dye $F_1$

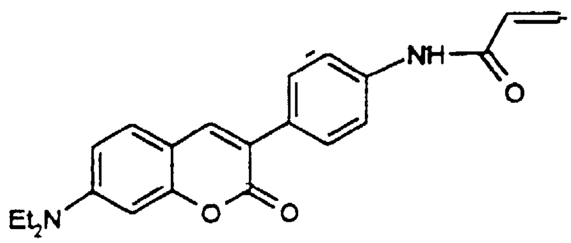

(II)

as second layer, and of a polylysine derivatized with digitoxigenin as receptor as top layer, labelling of anti-digoxin immunoglobulin G with tetramethylrhodamine isothiocyanate as dye $F_2$ in solution, immersion of the sensor in the solution, subsequent immersion in a digitoxin-containing solution, and measurement of the decrease in the fluorescence intensity from dye $F_2$ or the increase in the fluorescence intensity from $F_1$ or the change in the ratio of the two.

**11.** Process for the production of an optical biosensor according to Claim 1 or 2, characterized in that a plurality of layers is applied to an optionally transparent substrate by successive alternating immersion of the substrate in solutions of polyanionic and polycationic compounds, the substrate is washed and dried after each layer application, in which one of the top polyion layers contains a dye $F_1$ capable of fluorescence chemically bonded, and the top layer contains a receptor covalently or ionically bonded.

**12.** Use of the optical biosensor according to Claim 1 or 2 for detecting biochemically active compounds.

## Revendications

1. Biocapteur optique en phase solide apte à être marqué avec un colorant $F_1$ et avec une molécule réceptrice pour la détection de molécules d'analytes en phase liquide, apte à être marquée avec un colorant $F_2$ en utilisant le transfert d'énergie de Förster entre $F_1$ et $F_2$, qui est constitué par

   a) un support éventuellement transparent,

   b) une multicouche superposée à ce dernier,

   c) qui contient dans la couche supérieure ou dans une des couches supérieures, le fluorochrome $F_1$ à titre de colorant donneur, à l'état lié par voie chimique, et

   d) un anticorps ou un antigène à titre de récepteur, lié par voie covalente ou par voie ionique à la couche supérieure,

   e) auquel peut venir se lier un antigène, respectivement un anticorps à titre de molécule d'analyte, qui a été marqué à son tour avec le fluorochrome $F_2$, les molécules de colorant venant se placer avec un écartement qui permet un transfert d'énergie de Förster exempt de radiation, caractérisé en ce qu'on met en oeuvre, à titre de multicouche b), plusieurs couches alternantes sous forme de polyanions et de polycations, et en ce que la concentration de la molécule d'analyte liée est mesurée en fonction de l'augmentation de l'intensité de fluorescence relative de $F_2$, respectivement de la diminution de l'intensité de fluorescence de $F_1$ ou encore de la modification du rapport des deux intensités.

2. Biocapteur optique selon la revendication 1, dans lequel la molécule d'analyte e) déplace de la couche supérieure une molécule, qui n'est pas l'analyte, marquée avec le fluorochrome $F_2$ et liée à la couche supérieure, la concentration de la molécule d'analyte liée en définitive étant mesurée en fonction de la diminution de l'intensité de fluorescence relative de F2, respectivement de l'augmentation de l'intensité de fluorescence de $F_1$, ou encore de la modification du rapport des deux intensités.

3. Biocapteur optique selon la revendication 1 ou 2, dans lequel on met en oeuvre, à titre de matière de support, a) du verre flotté, du verre quartzeux, du silicium, du polyester, des polymères vinyliques ou du polycarbonate.

4. Biocapteur optique selon la revendication 1 ou 2, dans lequel, en ce qui concerne les composés polyanioniques, il s'agit de l'acide polyglutamique, de l'acide polystyrène-sulfonique, du sulfate de dextrane ou d'un copolymère d'acide styrènesulfonique ou d'autres acides vinylsulfoniques et d'un dérivé vinylique d'un fluorochrome.

5. Biocapteur optique selon la revendication 4, dans lequel, en ce qui concerne les composés polyanioniques, il s'agit de copolymères de styrènesulfonate ou de méthacrylate de sulfopropyle et de l'acrylate d'un colorant de coumarine.

6. Biocapteur optique selon la revendication 1 ou 2, dans lequel, en ce qui concerne les composés polycationiques, il s'agit de la polylysine, de la polyallylamine, de la polyvinylpyridine, du chitosane ou de leurs sels d'ammonium quaternisés par perméthylation ou encore du DEAE-dextrane.

7. Biocapteur optique selon la revendication 6, dans lequel, en ce qui concerne les composés polycationiques, il s'agit de composés polyamino modifiés en partie avec du fluorescéine-isothiocyanate ou avec un autre colorant réactif, tels que la polylysine ou la polyallylamine.

8. Biocapteur optique selon la revendication 1 ou 2, caractérisé en ce qu'on met en oeuvre, pour une paire de colorants $F_1$ et $F_2$ appropriés pour un transfert de Förster,

   des dérivés de fluorescéine et de la tétraméthylrhodamine,

   des dérivés de fluorescéine et du rouge du Texas,

   de la coumarine de formule I et de la tétraméthylrhodamine

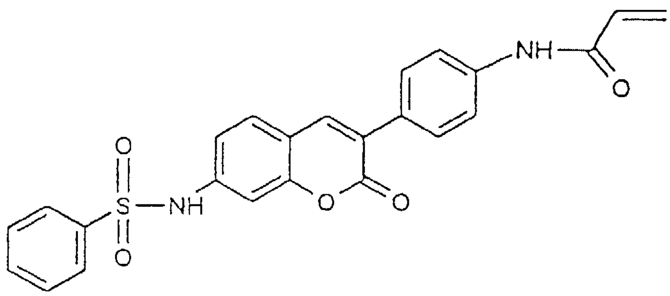

(I)

ou

de la coumarine de formule II et de la tétraméthylrhodamine

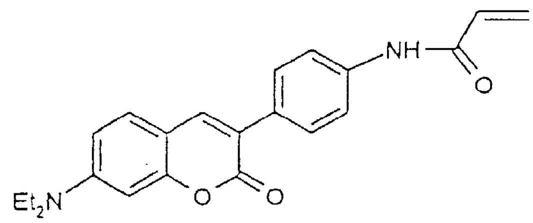

(II)

9. Biocapteur optique selon la revendication 8, dans lequel, à titre de paire de colorants $F_1$ et $F_2$, on met en oeuvre de la coumarine de formule I et de la triméthylrhodamine ou de la coumarine de formule II et de la tétraméthylrhodamine.

10. Procédé pour la détermination de la teneur en digoxine à titre de molécule d'analyte à l'aide d'un biocapteur selon la revendication 1, caractérisé en ce qu'on applique, sur un support en verre, à titre de première couche, une polylysine, à titre de deuxième couche, un copolymère de sulfopropylméthacrylate de potassium et d'une coumarine de formule II à titre de colorant $F_1$

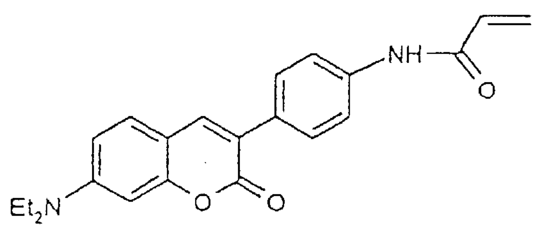

(II)

et, à titre de couche supérieure, une polylysine modifiée avec de la digitoxigénine à titre de récepteur, on marque l'immunoglobuline G anti-digoxine avec du tétraméthylrhodamine-isothiocyanate à titre de colorant $F_2$ en solution, on plonge le capteur dans la solution, on le plonge ensuite dans une solution contenant de la digitoxine et on mesure la diminution de l'intensité de la fluorescence émise par le colorant $F_2$, respectivement l'augmentation de l'intensité de la fluorescence émise par le colorant $F_1$ ou encore la modification du rapport des deux.

11. Procédé pour la préparation d'un biocapteur optique selon la revendication 1 ou 2, caractérisé en ce qu'on applique plusieurs couches sur un support éventuellement transparent, par immersions successives en alternance du support dans des solutions de composés polyanioniques et polycationiques, on lave et on sèche le support après chaque application de couche, dans lequel une des couches supérieures à teneur polyionique contient un fluorochrome $F_1$ lié par voie chimique et la couche supérieure contient un récepteur lié par voie covalente ou par voie ionique.

12. Utilisation du biocapteur optique selon la revendication 1 ou 2, pour la détection de composés à activité biochimique.

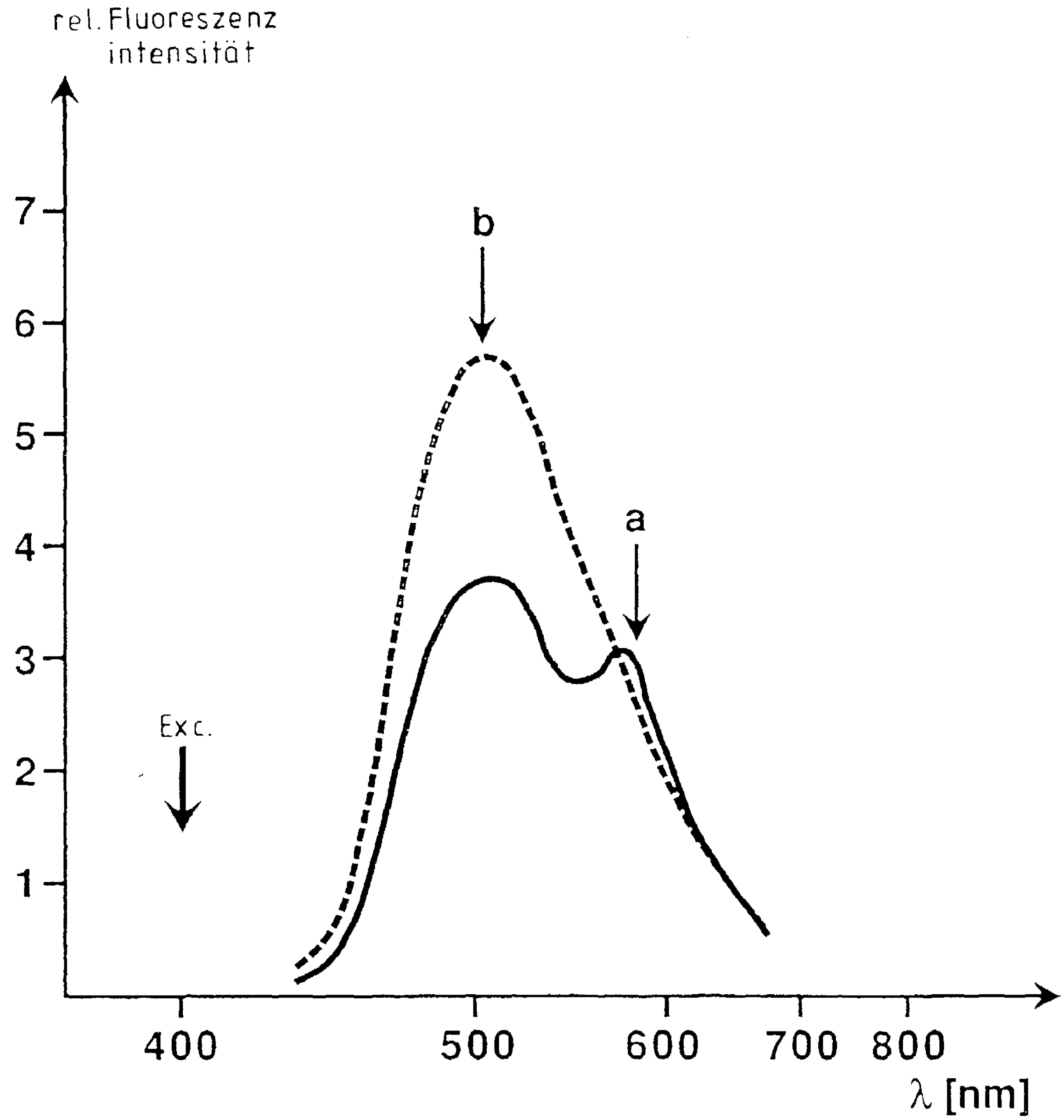
rel. Fluoreszenz intensität
7
6
5
4
3
2
1
b
a
Exc.
400
500
600
700
800
λ [nm]

FIG. 1